(19) Europäisches Patentamt
European Patent Office
Office européen des brevets

(11) Veröffentlichungsnummer: **0 169 458**
**B1**

(12) # EUROPÄISCHE PATENTSCHRIFT

(45) Veröffentlichungstag der Patentschrift: **01.06.88**

(51) Int. Cl.⁴: **G 03 C 7/36** // C07D405/04

(21) Anmeldenummer: **85108703.1**

(22) Anmeldetag: **12.07.85**

(54) **Farbfotografisches Aufzeichnungsmaterial mit einem Gelb-DIR-Kuppler.**

(30) Priorität: **24.07.84 DE 3427235**

(43) Veröffentlichungstag der Anmeldung: **29.01.86 Patentblatt 86/5**

(45) Bekanntmachung des Hinweises auf die Patenterteilung: **01.06.88 Patentblatt 88/22**

(84) Benannte Vertragsstaaten: **BE DE FR GB IT**

(56) Entgegenhaltungen:
**CH - A - 621 417**
**FR - A - 2 404 871**

(73) Patentinhaber: **Agfa-Gevaert AG, Patentabteilung, D-5090 Leverkusen 1 (DE)**

(72) Erfinder: **Öhlschläger, Hans, Dr., Am Katterbach 34, D-5060 Bergisch Gladbach 2 (DE)**
Erfinder: **Griesel, Ulrich, Dr., Böcklerstrasse 7, D-5090 Leverkusen 3 (DE)**
Erfinder: **Odenwälder, Heinrich, Dr., Am Arenzberg 37, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein farbfotografisches Aufzeichnungsmaterial mit mindestens einer lichtempfindlichen Silberhalogenidemulsionsschicht, die einen Gelbkuppler enthält, der bei Farbentwicklung einen Entwicklungsinhibitor freisetzt.

Es ist bekannt, die chromogene Entwicklung in Gegenwart von Verbindungen durchzuführen, die bei der Entwicklung bildmässig diffusionsfähige Substanzen freisetzen, die die Entwicklung von Silberhalogenid zu inhibieren vermögen. Derartige Verbindungen werden als sogenannte DIR-Verbindungen (DIR = development inhibitor releasing) bezeichnet. Bei der DIR-Verbindung kann es sich um solche handeln, die unter Abspaltung eines Inhibitorrestes mit dem Oxidationsprodukt eines Farbentwicklers zu einen Farbstoff reagieren (DIR-Kuppler), oder auch um solche, die den Inhibitor freisetzen ohne gleichzeitig einen Farbstoff zu bilden. Letztere werden auch als DIR-Verbindungen im engeren Sinne bezeichnet.

DIR-Kuppler sind beispielsweise bekannt aus US-A-3 148 061, US-A-3 227 554, US-A-3 615 506 und US-A-3 617 291.

Bei den freigesetzten Entwicklungsinhibitoren handelt es sich in der Regel um heterocyclische Mercaptoverbindungen oder um Derivate des Benzoltriazols. Hinsichtlich der im wesentlichen farblos kuppelnden DIR-Verbindungen sei beispielsweise verwiesen auf US-A-3 632 345, DE-A-2 359 295 und DE-A-2 540 959. Durch Anwendung von DIR-Verbindungen kann eine Vielzahl von fotografischen, die Bildqualität beeinflussenden Effektes bewirkt werden. Solche Effekte sind beispielsweise die Erniedrigung der Gradation, die Erzielung eines feineren Farbkorns, die Verbesserung der Schärfe durch den sogenannten Kanteneffekt und die Verbesserung der Farbreinheit und der Farbbrillanz durch sogenannte Interimageeffekte. Zu verweisen ist beispielsweise auf die Publikation «Development-Inhibitor-Releasing (DIR) Couplers in Color Photography» von C.R. Barr, J.R. Thirtle und P.W. Vittum, Photographie Science and Engineering 13, 74 (1969).

Die farblos kuppelnden DIR-Verbindungen haben vor den farbig kuppelnden DIR-Kupplern den Vorteil, dass sie universell einsetzbar sind, so dass die gleiche Verbindung ohne Rücksicht auf die zu erzeugende Farbe in allen lichtempfindlichen Schichten eines farbfotographischen Aufzeichnungsmaterials verwendet werden kann. DIR-Kuppler können dagegen wegen der aus ihnen erzeugten Farbe, meist nur in einem Teil der lichtempfindlichen Schichten verwendet werden, falls nicht die auf sie zurückführende Farbnebendichte in den anderen Schichten tolerierbar ist. Diesem Vorteil der DIR-Verbindungen steht als Nachteil gegenüber, dass sie im allgemeinen weniger reaktiv sind als die DIR-Kuppler. In der Praxis hat man sich daher meist darauf beschränkt, DIR-Kuppler zu verwenden und zwar notfalls zwei oder mehrere verschiedene im gleichen Aufzeichnungsmaterial, wobei den unterschiedlich spektral sensibilisierten Schichten verschiedene DIR-Kuppler nach Massgabe der aus den letzteren erzeugten Farbe zuzuordnen waren.

In DE-A-2 842 063 sind DIR-Kuppler beschrieben, die sich von Gelbkupplern ableiten und als abspaltbaren Inhibitorrest einen 3-Alkylthio-1,2,4-triazolylrest enthalten.

Bei Verwendung der dort beschriebenen DIR-Kuppler in einer blauempfindlichen Silberhalogenidemulsionsschicht kann zwar die Farbgradation in dieser Schicht beträchtlich verringert werden, jedoch ist die Auswirkung auf benachbarte Silberhalogenidschichten, insbesondere auf eine benachbarte grün- und/oder rotempfindliche Silberhalogenidemulsionsschicht vergleichsweise gering. Mit den bekannten DIR-Kupplern lassen sich daher nur geringe Interimageeffekte erzeugen.

Der Erfindung liegt die Aufgabe zugrunde ein farbfotografisches Aufzeichnungsmaterial anzugeben, das Gelb-DIR-Kuppler enthält, mit denen vergleichsweise hohe Interimageeffekte erzeugte werden können.

Gegenstand der Erfindung ist ein farbfotografisches Aufzeichnungsmaterial mit mindestens einer lichtempfindlichen Silberhalogenidemulsionsschicht und einem dieser zugeordneten DIR-Kuppler, der an die Kupplungsstelle eines Gelbkupplers gebunden einen abspaltbaren 1,2,4-Triazolylrest trägt, dadurch gekennzeichnet, dass der DIR-Kuppler der folgenden Formel I entspricht

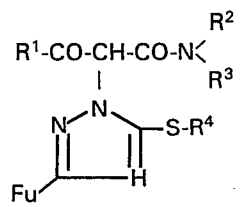

(I)

worin bedeuten

$R^1$ geradkettiges oder verzweigtes Alkyl, gegebenenfalls substituiertes Aryl oder $-NR^5R^6$

$R^2$, $R^5$ H oder Alkyl mit 1 bis 3 C-Atomen,

$R^3$, $R^6$ Alkyl mit 1 bis 18 C-Atomen oder gegebenenfalls substituiertes Aryl,

$R^4$ Alkyl mit 5 bis 12 C-Atomen, und

Fu einen gegebenenfalls substituierten Furylrest.

Ein durch $R^1$ dargestellter Alkylrest enthält 1 bis 18 C-Atome; er kann geradkettig oder verzweigt sein und ist vorzugsweise ein Tertiäralkylrest. Beispiele hierfür sind Methyl, Ethyl, Propyl, Isopropyl, n-Butyl, t-Butyl, t-Amyl, 1,1,3,3-Tetramethylbutyl. Der Alkylrest kann auch cyclische und bicyclische Reste umfassen, wie beispielsweise Norbornyl und Adamantyl.

Ein durch $R^1$ bzw. $R^3$ dargestellter Arylrest ist insbesondere ein Phenylrest, der substituiert sein kann, z.B. durch Alkyl, Alkoxy, Halogen, Carbamoyl, Sulfamoyl oder Acylamino, wobei der Acylrest abgeleitet ist von aliphatischen oder aromatischen Carbonsäure oder Sulfonsäuren, oder von Kohlensäuremonoestern oder Carbaminsäuren.

Ein durch $R^4$ dargestellter Alkylrest kann geradkettig oder verzweigt sein und gegebenenfalls durch Aryl substituiert (z.B. Benzyl).

Fu stellt einen über die 2- pder 3-Stellung angeknüpften Furylrest dar, der weitere Substituenten

tragen kann, z.B. Furyl-2, 2-Methylfuryl-3, 5-Nitrofuryl-2.

Nach der Darstellung der Formel I ist der 1,2,4-Triazolring mit einem seiner beiden benachbarten Ringstickstoffatome an die Kupplungsstelle des Gelbkupplers geknüpft. Da jedoch bis heute nicht völlig geklärt ist, ob dies tatsächlich der Realität entspricht, soll Formel I sich auch auf die entsprechenden Isomeren beziehen, in denen der 1,2,4-Triazolring über ein beliebiges anderes Ringstickstoffatom an die Kupplungsstelle gebunden ist.

Beispiele für geeignete Gelb-DIR-Kuppler gemäss der vorliegenden Erfindung sind in folgender Tabelle 1 angegeben ($R^2$ = H; Fu = 2-Furyl).

Tabelle 1

| Verbindung | $R^1$ | $R^3$ | $R^4$ |
|---|---|---|---|
| 1 | t-$C_4H_9$- | 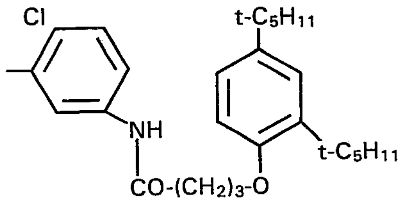 | -n-$C_6H_{13}$ |
| 2 | » | » | -n-$C_8H_{17}$ |
| 3 | » | » | 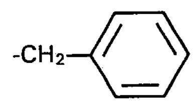 |
| 4 | 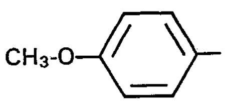 | 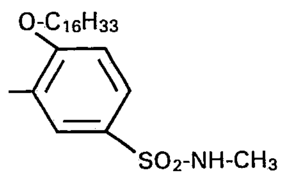 | -n-$C_6H_{13}$ |
| 5 | 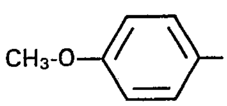 | 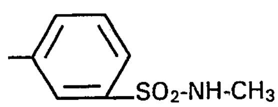 | 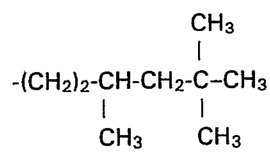 |
| 6 | 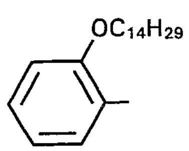 | 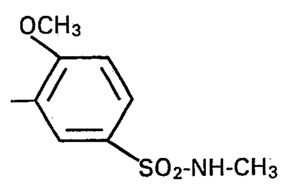 | -n-$C_6H_{13}$ |
| 7 | 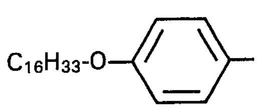 | 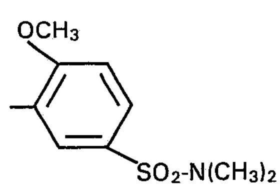 | -n-$C_6H_{13}$ |
| 8 | » | » | -n-$C_{10}H_{21}$ |

Tabelle 1 (Fortsetzung)

| Verbindung | $R^1$ | $R^3$ | $R^4$ |
|---|---|---|---|
| 9 | 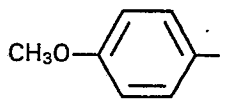 | 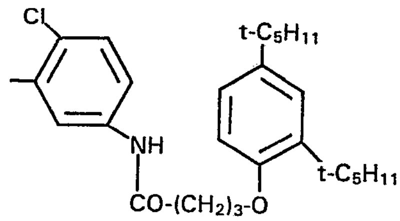 | $-n-C_6H_{13}$ |
| 10 | 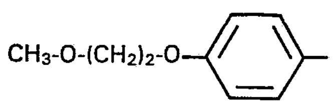 | 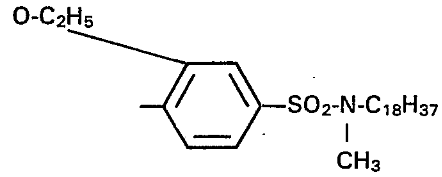 | $-n-C_6H_{13}$ |
| 11 | 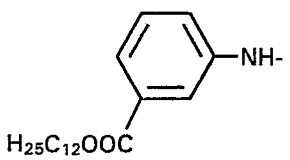 | 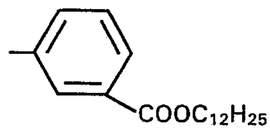 | $-n-C_6H_{13}$ |

Die erfindungsgemässen DIR-Kuppler der Formel I werden leicht erhalten durch Kondensation der bekannten α-Halogenacylacetanilide der Formel II

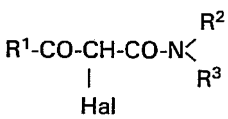 (II)

worin $R^1$ bis $R^3$ die bereits angegebene Bedeutung haben und Hal ein Halogenatom, insbesondere Chlor oder Brom bedeutet, mit Triazolen der Formel III

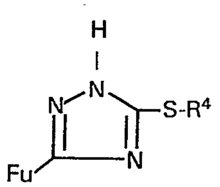 (III)

worin $R^4$ und Fu die angegebene Bedeutung haben. Die Umsetzung wird dabei vorteilhaft in einem organischen Lösungsmittel wie Dimethylformamid, Acetonitril oder Aceton in Gegenwart einer Base wie Triethylamin oder Ätzkali durchgeführt.

Die Triazole der Formel III sind ihrerseits zugänglich durch Umsetzung der entsprechenden 3-Mercapto-1,2,4-triazole mit geeigneten Alkyl- oder Aralkylhalogeniden.

Da die Triazole der Formel III in verschiedenen tautomeren Formen auftreten können und dem entsprechenden Azeniation demgemäss verschiedene mesomere Grenzstrukturen zugeordnet werden können, ist bei der Kondensation die Verknüpfung mit dem C-Atom der Kupplungsstelle über jedes der vorhandenen Ringstickstoffatome denkbar, so dass das Auftreten entsprechender Isomerer erklärbar ist. Diese Isomerie hat jedoch keinen Einfluss auf die Gebrauchseigenschaften der erfindungsgemässen DIR-Kuppler, so dass sich ein Eingehen auf die Struktur der möglichen Isomeren erübrigt.

Die Herstellung der erfindungsgemässen DIR-Kuppler wird im folgenden am Beispiel des DIR-Kupplers Verbindung 4 näher erläuert.

*Verbindung 4*

a.) 3-Hexylthio-5-(furyl-2)-1,2,4-triazol

500 g 3-Mercapto-5-(furyl-2)-1,2,4-triazol wurden mit 530 g Hexylbromid und 168 g Ätzkali in 3000 ml Methanol 8 h unter Rückfluss erhitzt. Das während der Reaktion ausgefallene KBr wurde abgesaugt, ca. 2500 ml Methanol wurden abdestilliert und der Rückstand wurde mit Wasser verrührt. Das dabei ausfallende Produkt wurde abgesaugt und getrocknet.

Ausbeute: 722 g

Schmelzpunkt 96°C (nach Umkristallisation aus Cyclohexan).

b.) *Verbindung 4*

356 g α-(4-Methoxybenzoyl)-α-chlor-2-hexadecyloxy-5-N-methylsulfamoylacetanilid wurden mit 140 g des unter a) hergestellten Triazols in 1500 ml Acetonitril mit 63 g Ätzkali 15 h bei Raumtemperatur gerührt. Die Reaktionslösung wurde auf 1500 ml 10%ige Salzsäure gegossen und das ölig anfallende Produkt in 500 ml Toluol aufgenommen. Die Toluollösung wurde eingeengt, der ölige Rückstand wurde in 1300 ml Cyclohexan eingegossen. Das dabei auskristallisierende Produkt wurde abgesaugt und an der Luft getrocknet.

Ausbeute: 300 g.
Schmelzpunkt: 63-65°C.

Die Verbindungen der vorliegenden Erfindung eignen sich für die Verwendung als Gelb-DIR-Kuppler in farbfotografischen, insbesondere mehrschichtigen Aufzeichnungsmaterialien. Als Gelbkuppler werden sie bevorzugt in oder zugeordnet zu einer lichtempfindlichen Silberhalogenidemulsionsschicht mit einer überwiegenden Empfindlichkeit für den blauen Spektralbereich des sichtbaren Lichtes verwendet. Der besondere Vorteil der erfindungsgemässen Gelb-DIR-Kuppler, nämlich eine vergleichsweise geringe Entwicklungsinhibierung in der Schicht, der eine solche Verbindung zugeordnet ist, neben einer vergleichsweise hohen Entwicklungsinhibierung in benachbarten nicht zugeordneten Schichten, kommt naturgemäss besonders dann zum Tragen, wenn es sich um ein mehrschichtiges farbfotografisches Aufzeichnungsmaterial handelt, das neben einer überwiegend blauempfindlichen Silberhalogenidemulsionsschicht weitere lichtempfindliche Silberhalogenidemulsionsschichten enthält mit überwiegender Empfindlichkeit für den grünen bzw. roten Spektralbereich des sichtbaren Lichtes. Fortschrittliche farbfotografische Aufzeichnungsmaterialien enthalten für jeden der drei Spektralbereiche Blau, Grün, Rot mindestens je eine lichtempfindliche Silberhalogenidemulsionsschichteneinheit mit zugeordneten Farbkupplern. Jede der genannten Silberhalogenidemulsionsschichteneinheiten kann aus einer oder auch aus mehreren Silberhalogenidemulsionsteilschichten bestehen, die im letzteren Fall in der Regel für den gleichen Spektralbereich sensibilisiert sind, sich aber in der Allgemeinempfindlichkeit, in der Art des verwendeten Silberhalogenids, in der Art des verwendeten Farbkupplers, in dem Mengenverhältnis Kuppler-zu-Silber sowie gegebenenfalls in weiteren physikalischen oder chemischen Parametern unterscheiden können. Die einzelnen Teilschichten gleicher Spektralempfindlichkeit können auch räumlich zu Silberhalogenidschichteneinheiten zusammen gefasst sein oder aber so angeordnet sein, dass eine (Teil-)schicht mit einer Empfindlichkeit gegen einen Teil des sichtbaren Spektrums sich zwischen zwei Teilschichten einer Schichteneinheit mit einer anderen Spektralempfindlichkeit befindet. Zwischen zwei Schichten unterschiedlicher Spektralempfindlichkeit ist zweckmässigerweise eine nicht lichtempfindliche Zwischen- oder Trennschicht angeordnet.

Den einzelnen Silberhalogenidemulsionsschichteneinheiten ist wie bereits erwähnt mindestens ein Farbkuppler zugeordnet. Unter «Zuordnung» bzw. «zugeordnet» ist dabei zu verstehen, dass die gegenseitige Anordnung von Silberhalogenidemulsionsschichteneinheit und Farbkuppler von solcher Art ist, dass bei der Entwicklung eine Wechselwirkung zwischen ihnen möglich ist, die eine bildgemässe Übereinstimmung zwischen gebildetem Silberbild und gebildetem Farbbild zulässt. Der betreffende Farbkuppler kann hierbei zweckmässigerweise in eine oder mehrere der vorhandenen Silberhalogenidemulsionsteilschichten einer Silberhalogenidemulsionsschichteneinheit oder auch in eine hierzu benachbarte nicht lichtempfindliche Bindemittelschicht eingelagert sein.

Die gegebenenfalls den zwei oder mehr Teilschichten einer Silberhalogenidemulsionsschichteneinheit zugeordneten Farbkuppler brauchen nicht notwendigerweise identisch zu sein. Sie sollen lediglich bei der Farbentwicklung die gleiche Farbe ergeben, normalerweise eine Farbe, die komplementär ist zu der Farbe des Lichtes, gegen das die lichtempfindlichen Silberhalogenidemulsionsschichten überwiegend empfindlich sind. Den rotempfindlichen Silberhalogenidemulsionsschichten ist folglich mindestens ein Farbkuppler zur Erzeugung des blaugrünen Teilfarbenbildes zugeordnet, in der Regel ein Kuppler vom Phenol- oder α-Naphtholtyp. Den grünempfindlichen Silberhalogenidemulsionsschichten ist mindestens ein Farbkuppler zur Erzeugung des purpurnen Teilfarbenbildes zugeordnet, wobei üblicherweise Farbkuppler vom Typ des 5-Pyrazolons oder des Indazolons Verwendung finden. Den blauempfindlichen Silberhalogenidemulsionsschichten ist schliesslich mindestens ein Farbkuppler zur Erzegung des gelben Teilfarbenbildes zugeordnet, in der Regel ein Farbkuppler mit einer offenkettigen Ketomethylengruppierung. Farbkuppler dieser Arten sind in grosser Zahl bekannt und in einer Vielzahl von Patentschriften beschrieben. Beispielhaft sei hier auf die Veröffentlichungen «Farbkuppler» von W. PELZ in «Mitteilungen aus den Forschungslaboratorien der Agfa, Leverkusen/München», Band III, Seite 111 (1961) und K. VENKATAMARAN in «The Chemistry of Synthetic Dyes», Vol. 4, 341 bis 387, Academic Press (1971), verwiesen.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, dass sie in der Kupplungsstelle einen Substituenten enthalten, der bei der Kupplung abgespalten wird. Zu den gemäss der vorliegenden Erfindung verwendbaren 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls gemäss der Erfindung zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalentkupplern sind auch die bekannten Weisskuppler zu rechnen, die jedoch bei Reaktion mit Farbentwickleroxidationsprodukten keinen Farbstoff ergeben, sowie ferner die bekannten DIR-Kuppler, bei denen es sich um Kuppler handelt, die in der Kupplungsstelle einen abspaltbaren Rest enthalten, der bei Reaktion mit Farbentwickleroxidationsprodukten als diffundierender Entwicklungsinhibitor in Freiheit gesetzt wird. Zu dieser Kategorie von 2-Äquivalentkupplern sind auch die Gelb-DIR-Kuppler der vorliegenden Erfindung zu rechnen.

Bei Bedarf können Farbkupplermischungen verwendet werden, um einen gewünschten Farbton oder eine gewünschte Reaktivität einzustellen. Beispielsweise können wasserlösliche Kuppler in Kombination mit hydrophoben wasserunlöslichen Kupplern verwendet werden.

Die Farbkuppler wie auch die Gelb-DIR-Kuppler

der vorliegenden Erfindung sind im allgemeinen diffusionsfest in die jeweilige Schicht eingelagert, wenngleich es in manchen Fällen auch vorteilhaft sein kann, Farbkuppler mit einer gewissen begrenzten Diffusionsneigung zu verwenden. Von den aus den Farbkupplern bei der Entwicklung gebildeten Bildfarbstoffen wird in der Regel zu fordern sein, dass sie in den Schichten nicht mehr zu diffundieren vermögen.

Obgleich die Erfindung vorstehend anhand solcher Aufzeichnungsmaterialien erläutert wurde, bei denen die Farbbilderzeugung durch chromogene Entwicklung unter Verwendung von Farbkupplern erfolgt, ist die Erfindung hierauf keinesfalls beschränkt. Die Einwirkung auf die Gradation der Schicht, in der die erfindungsgemässen Gelb-DIR-Kuppler enthalten sind, wie auch der benachbarten Schichten ist völlig unabhängig von der Art der im übrigen verwendeten Farbbildner. In gleicher Weise wie die erwähnten Farbkuppler können daher auch andere Arten von farbgebenden Verbindungen Verwendung finden, wie beispielsweise Entwicklerfarbstoffe oder Farbabspalter, wie sie in farbdiffusionsfotografischen Verfahren verwendet werden. Voraussetzung ist jedoch in jedem Fall, dass die Entwicklung unter Bedingungen vorgenommen wird, die eine bildmässige Freisetzung eines diffusionsfähigen Entwicklungsinhibitors, hier eines 3-Alkylthio-5-furyl-1,2,4-triazols, nach Massgabe einer voraufgegangenen Belichtung ermöglichen. Das bedeutet, dass die erfindungsgemässen farbfotografischen Aufzeichnungsmaterialien in Gegenwart solcher Entwicklerverbindungen entwickelt werden, deren Oxidationsprodukte mit den erfindungsgemässen Gelb-DIR-Kuppler unter Freisetzung eines Entwicklungsinhibitors reagieren. Die üblicherweise verwendeten p-Phenylendiaminverbindungen mit einer freien primären Aminogruppe sind Beispiele solcher geeigneter Entwicklungsverbindungen.

Die einzusetzende Menge an erfindungsgemässem Gelb-DIR-Kuppler in der Schicht kann je nach Bedarf innerhalb weiter Grenzen variiert werden, z.B. zwischen $0.5 \cdot 10^{-4}$ und $50 \cdot 10^{-4}$ mol/m$^2$. Günstigere Ergebnisse werden beispielhaft erzielt bei einer Menge an erfindungsgemässen Gelb-DIR-Kupplern zwischen 1,0 und $5{,}0 \cdot 10^{-4}$ mol/m$^2$. Die Einarbeitung kann zweckmässigerweise unter Verwendung üblicher Ölbildner vorgenommen werden.

*Beispiel*

Zur Erläuterung der Erfindung wird ausgegangen von dem nachstehend beschriebenen Schichtaufbau. Hierzu werden die genannten Schichten in der angegebenen Reihenfolge auf einen mit einer Lichthofschutzschicht und einer Haftschicht versehenen transparenten Schichtträger aus Cellulosetriacetat aufgetragen. Die Mengenangaben beziehen sich jeweils auf 1 m$^2$. Für den Silberauftrag werden die entsprechenden Mengen $AgNO_3$ angegeben.

1. Eine weniger empfindliche rotempfindliche Schicht mit einer rotsensibilisierten Silberbromidiodidemulsion (5 Mol.-% AgI) aus 3,5 g $AgNO_3$ mit 600 mg eines Blaugrünkupplers der Formel

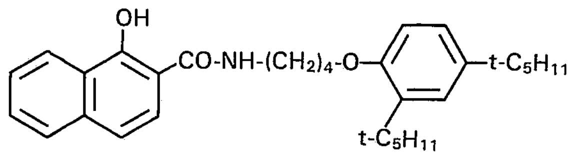

30 mg eines DIR-Kupplers der Formel

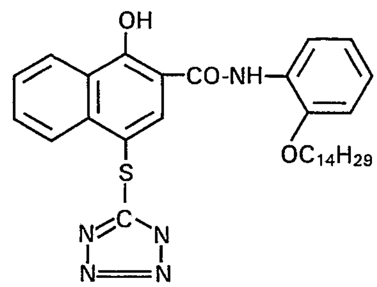

und 60 mg eines Maskenkupplers der Formel

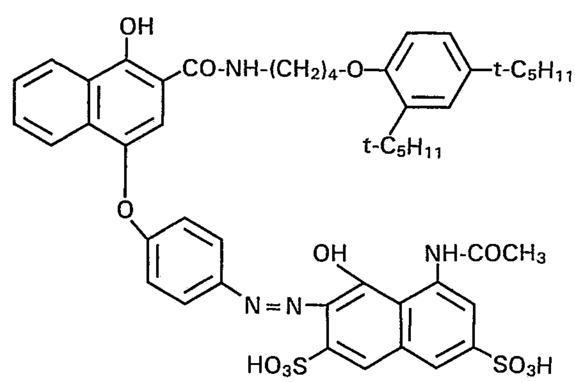

und 2,5 g Gelatine.

2. Eine empfindlichere rotempfindliche Schicht mit einer rotsensibilisierten Silberbromidiodidemulsion (8 Mol.-% AgI) aus 3,0 g $AgNO_3$ mit 200 mg des Blaugrünkupplers der Schicht 1 und 2,7 g Gelatine.

3. Eine Zwischenschicht mit 0,8 g Gelatine und 0,1 g 2,5-Diisooctylhydrochinon.

4. Eine weniger empfindliche grünempfindliche Schicht mit einer grünsensibilisierten Silberbromidiodidemulsion (6 Mol.-% AgI) aus 2,5 g $AgNO_3$ mit 800 mg eines Purpurkupplers der Formel

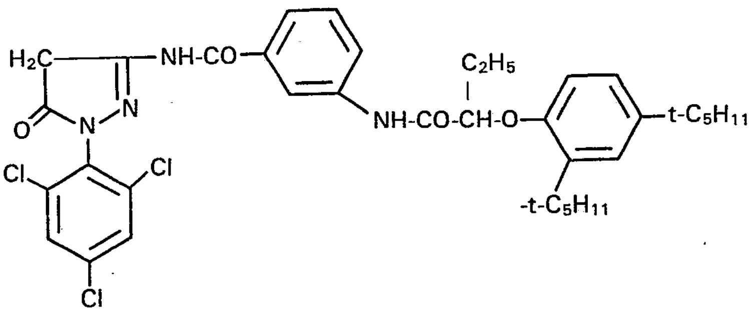

25 mg eines DIR-Kupplers der Formel

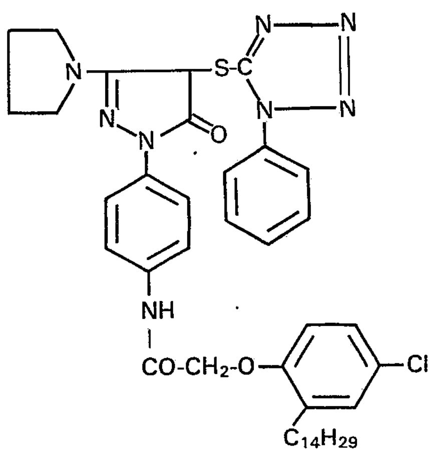

90 mg eines Maskenkupplers der Formel

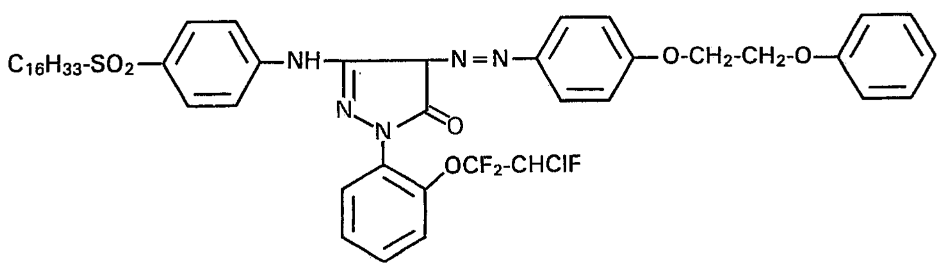

und 2,6 g Gelatine.

5. Eine empfindlichere grünempfindliche Schicht mit einer grünsensibilisierten Silberbromidiodidemulsion (10 Mol.-% AgI) aus 2,0 g $AgNO_3$ mit 250 mg des Purpurkupplers der Schicht 4 und 1,7 g Gelatine.

6. Eine Gelbfilterschicht mit kolloidalem Silber zur Erzeugung einer Gelbdichte von 0,7 und 0,6 g Gelatine.

7. Eine weniger empfindliche blauempfindliche Schicht mit einer Silberbromidiodidemulsion (5 Mol.-% AgI) aus 1,0 g $AgNO_3$ mit 700 mg eines Gelbkupplers der Formel

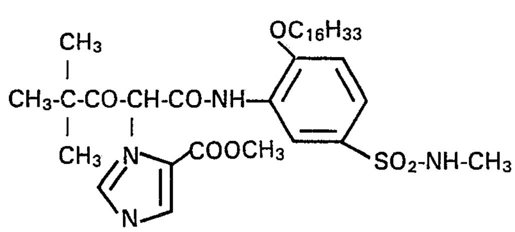

und einem DIR-Kuppler wie in der nachstehenden Tabelle 2 angegeben, sowie 0,9 g Gelatine.

8. Eine empfindlichere blauempfindliche Schicht mit einer blauempfindlichen Silberbromidiodidemulsion aus 1,3 g $AgNO_3$ mit 300 mg des Gelbkupplers der Schicht 7 und 0,7 g Gelatine.

9. Eine Deckschicht mit 0,6 g Gelatine.

Die Kuppler in den Schichten 1, 2, 4, 5, 7 und 8 sowie das Diisooctylhydrochinon in Schicht werden als Emulgat mit Trikresylphosphat (1 : 1) eingesetzt. Die in Schicht 7 verwendeten DIR-Kuppler sowie die damit in den drei Teilbildern Gelb, Purpur, Blaugrün ermittelten Inhibierungswerte sind der nachstehenden Tabelle 2 zu entnehmen. Der Inhibierungswert ist der hinter Blau-, Grün- bzw. Rotfilter gemessene Farbdichterückgang (△Db, △Dg, △Dr) eines Aufzeichnungsmaterials, das in der Schicht 7 einen der genannten DIR-Kuppler enthält, verglichen mit einem Aufzeichnungsmaterial, das in der Schicht 7 keinen DIR-Kuppler enthält, bezogen auf die absolute Dichte

$\triangle Dn = 1,5$ über der Minimaldichte Dmin. Zusätzlich ist für die hinter Grün- bzw. Rotfilter gemessenen Inhibierungswerte auch der betreffende Inhibierungsquotient

$$Qg = \frac{\triangle Dg}{\triangle Db} \quad ; \quad Qr = \frac{\triangle Dr}{\triangle Db}$$

angegeben.

Die Entwicklung wurde nach Aufbelichtung eines Graukeiles durchgeführt wie beschrieben in «The British Journal of Photography», 1974, Seiten 597 und 598.

Tabelle 2

| Verb. Nr. | Menge [$10^{-4}$mol] | Inhibierung △Db | △Dg | Qg | △Dr | Qr |
|---|---|---|---|---|---|---|
| 1 | 2,8 | 11 | 20 | 1,8 | 7 | 0,6 |
| 4 | 1,2 | 12 | 15 | 1,3 | 6 | 0,5 |
| 4 | 1,7 | 16 | 20 | 1,3 | 7 | 0,4 |
| 6 | 1,7 | 3 | 13 | 4,3 | 7 | 2,3 |
| 7 | 1,2 | 5 | 11 | 2,2 | 4 | 0,8 |
| 7 | 1,8 | 7 | 11 | 2,3 | 5 | 0,7 |
| 9 | 1,2 | 11 | 17 | 1,5 | 6 | 0,5 |
| 9 | 1,7 | 11 | 18 | 1,6 | 4 | 0,4 |
| 10 | 1,0 | 8 | 14 | 1,8 | 4 | 0,5 |
| 10 | 1,7 | 9 | 17 | 1,9 | 6 | 0,7 |
| A | 1,2 | 16 | 10 | 0,6 | 0 | 0 |
| A | 2,2 | 20 | 20 | 1,0 | 3 | 0,2 |
| B | 1,3 | 11 | 8 | 0,7 | 0 | 0 |
| B | 1,7 | 20 | 15 | 0,8 | 4 | 0,2 |

Tabelle 2 (Fortsetzung)

| Verb. Nr. | Menge [$10^{-4}$mol] | Inhibierung △Db | △Dg | Qg | △Dr | Qr |
|---|---|---|---|---|---|---|
| C | 1,4 | 28 | 20 | 0,7 | 7 | 0,3 |
| D | 1,3 | 25 | 18 | 0,7 | 8 | 0,3 |
| D | 1,8 | 35 | 25 | 0,7 | 8 | 0,2 |
| E | 1,8 | 0 | 3 | — | 0 | — |

Aus der Tabelle 2 ersichtlich, dass mit den in der weniger empfindlichen blauempfindlichen Schicht eingesetzten erfindungsgemässen Gelb-DIR-Kupplern eine beträchtliche Inhibierung der Entwicklung in den grünempfindlichen Schichten und damit eine beträchtliche Gradationserniedrigung des purpurnen Teilfarbenbildes erzielt wird, während die Inhibierung in der blauempfindlichen Schicht vergleichsweise gering ist. Dies äussert sich in Qg-Werten von deutlich über 1. Demgegenüber wirken sich die zum Vergleich herangezogenen Gelb-DIR-Kuppler A, B, C, D und E hauptsächlich auf die Gradation des gelben Teilfarbenbildes aus. Auch hinsichtlich der Einwirkung auf die weiter entfernten rotempfindlichen Schichten sind die erfindungsgemässen Gelb-DIR-Kuppler deutlich überlegen.

Entsprechend waren die Farbtrennung sowie die Körnigkeit und Schärfe des Materials bei Einsatz der erfindungsgemässen Verbindungen erheblich verbessert.

Die als Vergleich herangezogenen Gelb-DIR-Kuppler sind folgende:

Tabelle 3

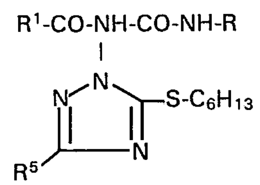

| Verbindung | $R^1$ | R | $R^5$ |
|---|---|---|---|
| A | t-$C_4H_9$ | 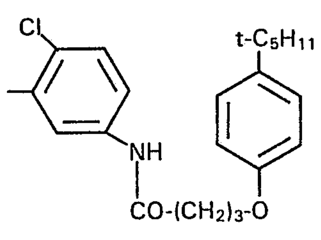 | H |
| | = Verbindung No. 202 aus DE-A-2 842 063 | | |
| B | 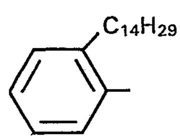 | 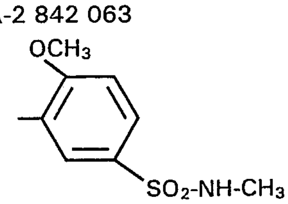 | $-NH_2$ |
| C | 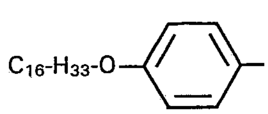 | 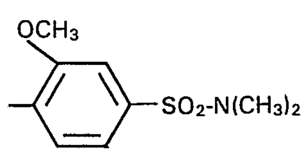 | H |

Tabelle 3 (Fortsetzung)

| Verbindung | $R^1$ | R | $R^5$ |
|---|---|---|---|
| D | 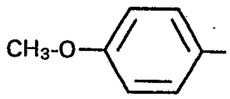 | 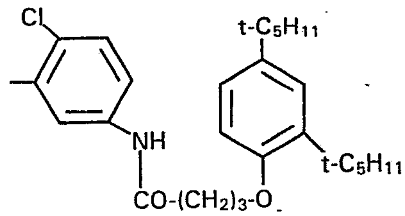 | H |
| E | 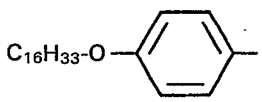 | 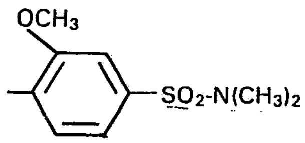 | 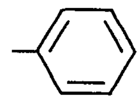 |

**Patentansprüche**

1. Farbfotografisches Aufzeichnungsmaterial mit mindestens einer lichtempfindlichen Silberhalogenidemulsionsschicht und einem dieser zugeordneten DIR-Kuppler, der an die Kupplungsstelle eines Gelbkupplers gebunden einen abspaltbaren 1,2,4-Triazolylrest trägt, dadurch gekennzeichnet, dass der DIR-Kuppler der folgenden Formel entspricht.

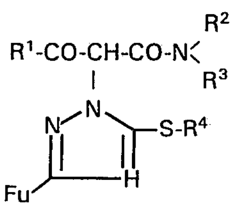

worin bedeuten
$R^1$ geradkettiges oder verzweigtes Alkyl, gegebenenfalls substituiertes Aryl oder $-NR^5R^6$
$R^2$, $R^5$ H oder Alkyl mit 1 bis 3 C-Atomen,
$R^3$, $R^6$ Alkyl mit 1 bis 18 C-Atomen oder gegebenenfalls substituiertes Aryl,
$R^4$ Alkyl mit 5 bis 12 C-Atomen, und
Fu einen gegebenenfalls substituierten Furylrest.

2. Aufzeichnungsmaterial nach Anspruch 1, dadurch gekennzeichnet, dass der DIR-Kuppler in einer überwiegend blauempfindlichen Silberhalogenidemulsionsschicht enthalten ist, und dass das Aufzeichnungsmaterial mindestens eine weitere überwiegend grünempfindliche oder überwiegend rotempfindliche Silberhalogenidemulsionsschicht enthält.

3. Farbfotografisches Aufzeichnungsmaterial mit mindestens einer überwiegend blauempfindlichen Silberhalogenidemulsionsschichteneinheit, der mindestens ein Gelbkuppler zugeordnet ist, einer überwiegend grünempfindlichen Silberhalogenidemulsionsschichteneinheit, der mindestens ein Purpurkuppler zugeordnet ist, und einer überwiegend rotempfindlichen Silberhalogenidemulsionsschichteneinheit, der mindestens ein Blaugrünkuppler zugeordnet ist, dadurch gekennzeichnet, dass mindestens eine Teilschicht der überwiegend blauempfindlichen Silberhalogenidemulsionsschichteneinheit eine Verbindung der folgenden Formel enthält:

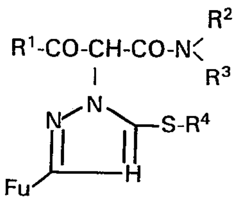

worin bedeuten
$R^1$ geradkettiges oder verzweigtes Alkyl, gegebenenfalls substituiertes Aryl oder $-NR^5R^6$
$R^2$, $R^5$ H oder Alkyl mit 1 bis 3 C-Atomen,
$R^3$, $R^6$ Alkyl mit 1 bis 18 C-Atomen oder gegebenenfalls substituiertes Aryl,
$R^4$ Alkyl mit 5 bis 12 C-Atomen, und
Fu einen gegebenenfalls substituierten Furylrest.

**Claims**

1. A colour photographic recording material having at least one light- sensitive silver halide emulsion layer and, associated with this layer, a DIR coupler which carries, in the coupling position, a yellow coupler attached to a 1,2,4-triazolyl group which can be split off, characterised in that the DIR coupler corresponds to the following formula:

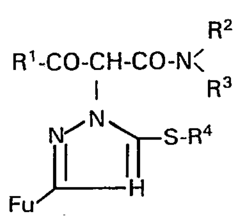

wherein

$R^1$ represents straight-chained or branched alkyl, optionally substituted aryl or $-NR^5R^6$

$R^2$, $R^5$ represent H or alkyl with 1 to 3 carbon atoms,

$R^3$, $R^6$ represent alkyl with 1 to 18 carbon atoms or optionally substituted aryl,

$R^4$ represents alkyl with 5 to 12 carbon atoms, and

Fu represents an optionally substituted furyl group.

2. A recording material according to claim 1, characterised in that the DIR coupler is contained in a predominantly blue-sensitive silver halide emulsion layer and in that the recording material contains at least one other silver halide emulsion layer, which is predominantly green-sensitive or predominantly red-sensitive.

3. A colour photographic recording material having at least one predominantly blue-sensitive silver halide emulsion layer unit with which at least one yellow coupler is associated, a predominantly green-sensitive silver halide emulsion layer unit with which at least one magenta coupler is associated, and a predominantly red-sensitive silver halide emulsion layer unit with which at least one cyan coupler is associated, characterised in that at least one partial layer of the predominantly blue-sensitive silver halide emulsion layer unit contains a compound corresponding to the following formula:

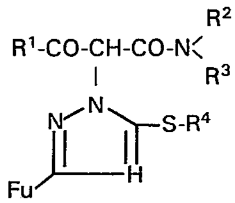

wherein

$R^1$ represents straight-chained or branched alkyl, optionally substituted aryl or $-NR^5R^6$

$R^2$, $R^5$ represent H or alkyl with 1 to 3 carbon atoms,

$R^3$, $R^6$ represent alkyl with 1 to 18 carbon atoms or optionally substituted aryl,

$R^4$ represents alkyl with 5 to 12 carbon atoms, and

Fu represents an optionally substituted furyl group.

**Revendications**

1. Matériau d'enregistrement de photographie en couleurs comportant au moins une couche d'émulsion photosensible à l'halogénure d'argent et un copulant DIR (= libérant un inhibiteur de développement) attribué à cette couche et comportant un radical 1,2,4-triazolyle dissociable lié à la position de copulation d'un copulant jaune, caractérisé en ce que le copulant DIR répond à la formule suivante:

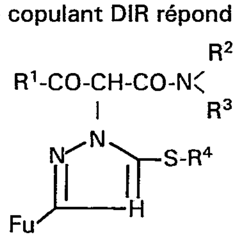

dans laquelle

$R^1$ représente un radical alkyle à chaîne droite ou ramifiée, un radical aryl éventuellement substitué ou un radical $-NR^5R^6$

$R^2$, $R^5$ représentent chacun H ou un radical alkyle contenant 1 à 3 atomes de carbone,

$R^3$, $R^6$ représentent chacun un radical alkyle contenant 1 à 18 atomes de carbone ou un radical aryle éventuellement substitué,

$R^4$ représente un radical alkyle contenant 5 à 12 atomes de carbone, et

Fu représente un radical furyle éventuellement substitué.

2. Matériau d'enregistrement selon la revendication 1, caractérisé en ce que le copulant DIR est contenu dans une couche d'émulsion à l'halogénure d'argent essentiellement sensible au bleu, et en ce que ce matériau d'enregistrement contient au moins une autre couche d'émulsion à l'halogénure d'argent essentiellement sensible au vert ou essentiellement sensible au rouge.

3. Matériau d'enregistrement de photographie en couleurs comportant au moins une unité de couches d'émulsions aux halogénures d'argent essentiellement sensible au bleu, à laquelle est attribué au moins un copulant jaune, une unité de couches d'émulsions aux halogénures d'argent essentiellement sensible au vert, à laquelle est attribué au moins un copulant magenta, ainsi qu'une unité de couches d'émulsions aux halogénures d'argent essentiellement sensible au rouge et à laquelle est attribué au moins un copulant bleu-vert, caractérisé en ce qu'au moins une couche partielle de l'unité de couches d'émulsions aux halogénures d'argent essentiellement sensible au bleu contient un composé répondant à la formule suivante:

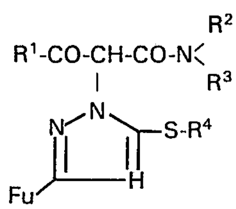

dans laquelle

$R^1$ représente un radical alkyle à chaîne droite ou ramifiée, un radical aryl, éventuellement substitué ou un radical $-NR^5R^6$

$R^2$, $R^5$ représentent chacun H ou un radical alkyle contenant 1 à 3 atomes de carbone,

$R^3$, $R^6$ représentent chacun un radical alkyle contenant 1 à 18 atomes de carbone ou un radical aryle éventuellement substitué,

$R^4$ représente un radical alkyle contenant 5 à 12 atomes de carbone, et

Fu représente un radical furyle éventuellement substitué.